# EUROPÄISCHE PATENTSCHRIFT

(11) **EP 4 120 182 B1**
(45) Veröffentlichungstag und Bekanntmachung des Hinweises auf die Patenterteilung: **27.08.2025**
(21) Anmeldenummer: 21184982.3
(22) Anmeldetag: 12.07.2021
(51) Int. Cl.: G06T 5/00, G06T 7/80, G06T 11/00

(54) **SELBSTKALIBRIERENDES DENTALES DVT UNTERSTÜTZT DURCH MASCHINELLES LERNEN**
SELF-CALIBRATING DENTAL DVT ASSISTED BY MACHINE LEARNING
TOMOGRAPHIE NUMÉRIQUE DE VOLUME DENTAIRE À ÉTALONNAGE AUTOMATIQUE ASSISTÉ PAR APPRENTISSAGE AUTOMATIQUE

(43) Veröffentlichungstag der Anmeldung: 18.01.2023
(73) Patentinhaber: DENTSPLY SIRONA Inc., York, PA 17401 (US); Sirona Dental Systems GmbH, 64625 Bensheim (DE)
(72) Erfinder: Maur, Susanne, 64625 Bensheim (DE); Hülsbusch, Markus, 64625 Bensheim (DE)
(74) Vertreter: Taor, Simon Edward William

(56) Entgegenhaltungen:
- US-A1- 2018 268 574

## Beschreibung

### TECHNISCHES GEBIET DER ERFINDUNG

Die vorliegende Erfindung betrifft ein System und Verfahren zur geometrischen Kalibrierung einer DVT-Aufnahme im dentalen Bereich zur Kompensation von Bewegungsartefakten im rekonstruierten Volumen.

### HINTERGRUND DER ERFINDUNG

In der Literatur gibt es Verfahren zur Autokalibrierung, welche aus einer gegebenen DVT Aufnahme, die zur Rekonstruktion oder Bildkorrektur erforderlichen geometrischen Parameter oder eine Bildkorrektur für das rekonstruierte Volumen schätzen. Es gibt konventionelle Verfahren, welche die Schätzung der geometrischen Parameter zum Beispiel durch eine Bewertung der Bildqualität oder Datentreue im rekonstruierten Volumen oder durch die Auswertung von Datenkonsistenzbedingungen vornehmen. Außerdem gibt es Korrekturverfahren auf Basis maschinellen Lernens (ML), welche zum Beispiel ein Artefakt-behaftetes Volumen in ein Artefakt-reduziertes Volumen transformieren. Die jeweiligen Vor- und Nachteile von den beiden Verfahrensarten werden im Folgenden weiter erläutert.

### Verfahren zur Parameterschätzung

Die Vorteile der konventionellen Verfahren zur Parameterschätzung sind, dass die Rohdaten ein wichtiger Bestandteil der Verarbeitungskette sind, somit kann die Rohdatendeckung, bzw. die Datentreue, geprüft und gesteuert werden. Die Änderungen an Bilddaten sind kontrollierbar, z.B. durch das Einführen von Randbedingungen.

Die Nachteile der konventionellen Verfahren zur Parameterschätzung sind, dass es sich um komplexe Optimierungsprobleme mit vielen zu schätzenden Parametern handelt, mit vielen Parameterabhängigkeiten und mit vielen lokalen Optima, welche nicht effizient gelöst werden können. Die Berechnung erfolgt meist iterativ. In der Praxis führt das zu langen Rechenzeiten und meist sub-optimalen Ergebnissen.

### ML Korrekturverfahren

Die Vorteile der ML Korrekturverfahren sind, dass es zu deutlich kürzere Rechenzeiten als bei konventionellen Verfahren zur Parameterschätzung kommt, da die Anwendung eines ML Korrekturverfahren einer direkten Berechnung entspricht und somit kein Optimierungsproblem enthält. Die globalere Betrachtung der Bilddaten und deren Zusammenhänge führt zu einer schnelleren und effizienteren Korrektur großer Fehler. Die Nachteile der ML Korrekturverfahren sind, dass Bild-zu-Bild Transformationen Daten erfinden können. Außerdem sind Änderungen an Bilddaten oder Parametern schwieriger kontrollierbar, weil Randbedingungen schwieriger in ML Korrekturverfahren zu integrieren sind.

Ein ML Korrekturverfahren, das aus dem Stand der Technik bekannt ist, wird im Folgenden aufgeführt: Jiang et al., "Wasserstein generative adversarial networks for motion artifact removal in dental CT imaging", Proc. SPIE 10948, Medical Imaging 2019: Physics of Medical Imaging, 2019.

Es wird auf das Dokument US2018268574A1 aus dem Stand der Technik verwiesen, in dem ein Verfahren zur Korrektur von Patientenbewegungen bei der computergestützten Kegelstrahltomographie offenbart wird.

### OFFENBARUNG DER ERFINDUNG

Ein Ziel der vorliegenden Erfindung ist die automatische geometrische Kalibrierung einer spezifischen Aufnahme, z.B. einer Patientenaufnahme. Somit können veraltete Gerätekalibrierungen aktualisiert oder Patienten-spezifische Anpassungen, wie zum Beispiel Patientenbewegung, korrigiert werden. Dies ermöglicht die Kompensation von Bewegungsartefakten im rekonstruierten Volumen.

Dieses Ziel wird durch das Verfahren nach Anspruch 1 erreicht. Die Gegenstände der abhängigen Ansprüche beziehen sich auf Weiterentwicklungen und bevorzugte Ausführungsformen.

Das erfindungsgemäße Verfahren dient zur geometrischen Kalibrierung einer DVT-Aufnahme gemäss Anspruch 1.

Ein wesentliches Merkmal dieser Erfindungsmeldung ist die Kombination der konventionellen Verfahren zur Parameterschätzung mit den ML Korrekturverfahren.

Ein vorteilhafter Effekt der vorliegenden Erfindung ist die Unterstützung des Verfahrens zur automatischen geometrischen Kalibrierung von dentalen Patientenaufnahmen durch Daten-basiertes Vorwissen, welches mittels ML Korrekturverfahren generiert wurde. Das ML Korrekturverfahren generiert ein Daten-basiertes Vorwissen, welches das konventionelle Verfahren zur Parameterschätzung stützt/stabilisiert/beschleunigt/steuert. Somit können die Vorteile beider Verfahren kombiniert und deren Nachteile minimiert werden.

Konkret kann das Ergebnis eines ML Korrekturverfahrens als initiale Schätzung für ein konventionelles Korrekturverfahren zur Parameterschätzung verwendet werden. Vorzugsweise wird das korrigierte Volumen in einem iterativen Verfahren als Regularisierung eines konventionellen Verfahrens eingesetzt. Oder es gibt an, wo und wie stark eine Änderung der Daten zu erwarten ist.

Dies ermöglicht es, die Rohdatendeckung/Datentreue im Gesamtverfahren zu erhalten und Randbedingungen zu integrieren. Auch wird der Geschwindigkeitsvorteil des ML Korrekturverfahren und seine globalere Betrachtung der Bilddaten genutzt. Das ML Korrekturverfahren setzt eine globale Betrachtung des Problems um und steuert somit in die Nähe des globalen Optimums. Das konventionelle Verfahren zur Parameterschätzung hingegen entspricht einer lokaleren Betrachtung des Problems und verbessert somit die Genauigkeit und Korrektheit/Datentreue des Ergebnisses.

### KURZBESCHREIBUNG DER ZEICHNUNGEN

In der nachfolgenden Beschreibung wird die vorliegende Erfindung anhand von beispielhaften Ausführungsformen und unter Bezugnahme auf die Zeichnungen näher erläutert, wobei
Abb.1 - zeigt ein Flussdiagram gemäß einer Ausführungsform der Erfindung;
Abb.2 - zeigt ein Flussdiagram gemäß einer weiteren Ausführungsform der Erfindung;
Abb.3 - zeigt ein Flussdiagram gemäß einer weiteren Ausführungsform der Erfindung;
Abb.4 - zeigt ein Flussdiagram gemäß einer weiteren Ausführungsform der Erfindung;
Abb.5 - zeigt ein computergestütztes DVT-System, auf dem das erfindungsgemäße Verfahren durchgeführt werden kann;
Abb. 6 - zeigt die örtliche Interpolation und Extrapolation der geometrischen Parameter im Volumen.

Die in den Zeichnungen gezeigten Referenznummern bezeichnen die unten aufgeführten Elemente, auf die in der nachfolgenden Beschreibung der beispielhaften Ausführungsformen Bezug genommen wird.
1. DVT-System
2. Röntgengerät
3. Röntgenstrahler
4. Röntgendetektor
5. Bedienungseinheit
6. Kopffixierung
7. Aufbiss
8. Rechner
9. Anzeige

a. Schädel des Pateinten
b. Gebogener Strahl
c. Erster selektierter Teilbereich des Volumen
d. Zweiter selektierter Teilbereich des Volumen

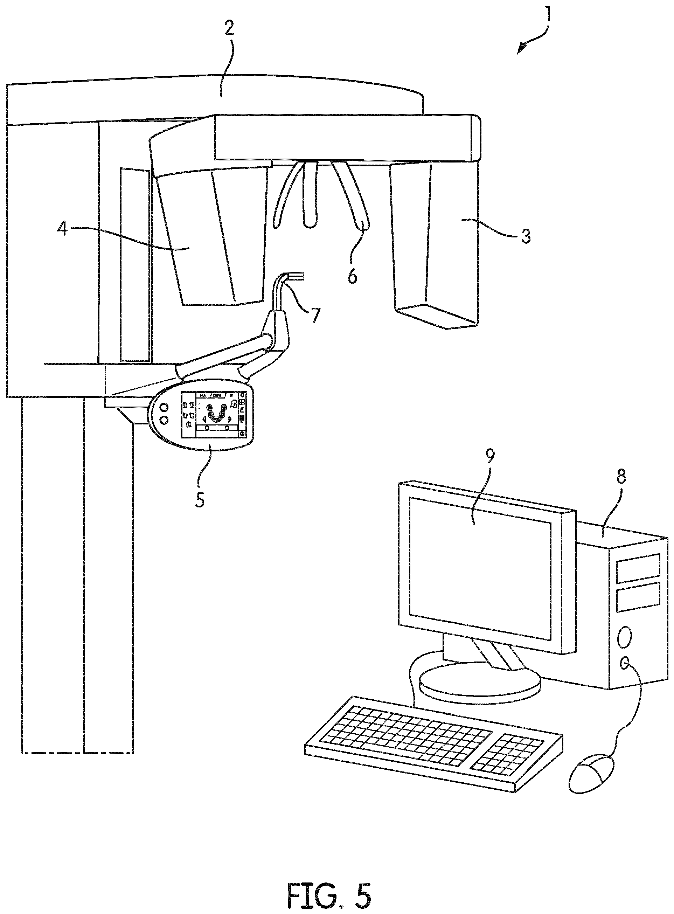

Das erfindungsgemäße Verfahren ist ein Computer implementierbares Verfahren, und kann auf einem computergestützten DVT-System (1) ausgeführt werden. Abb. 5 zeigt ein Ausführungsbespiel für ein DVT-System (1). Hierzu umfasst die vorliegende Erfindung auch ein Computerprogramm mit computerlesbarem Code. Das Computerprogramm kann auf einem Datenspeicher bereitgestellt werden. Das computergestützte DVT-System (1) umfasst ein Röntgengerät (2) zur Durchführung der Patientenaufnahme, wobei die Messdaten erzeugt werden. Die Messdaten umfassen das Sinogramm der Aufnahme bestehend aus Röntgenprojektionen. Das Röntgengerät (2) hat einen Röntgenstrahler (3) und Röntgendetektor (4), die während der Aufnahme um den Patentknopf gedreht werden. Der Patientenkopf wird mit dem Aufbiss (7) und der Kopffixierung (6) im Röntgengerät positioniert. Das computergestützte DVT-System (1) umfasst eine Bedienungseinheit (5), vorzugsweise einen Rechner (8) oder eine Recheneinheit, die mit dem Röntgengerät (2) verbunden werden kann, und vorzugsweise eine Anzeige (9), u.a. um die Datensätze zu visualisieren. Der Rechner (8) kann über ein lokales Netzwerk (nicht gezeigt) oder alternativ über das Internet mit dem Röntgengerät (2) verbunden werden. Der Rechner (8) kann Teil einer Cloud sein. Alternativ kann der Rechner (3) in das Röntgengerät (2) integriert werden. Die Berechnungen können alternativ in der Cloud stattfinden. Der Rechner (8) führt das Computerprogramm aus und liefert die Datensätze, u.a. auch für die Visulisierung auf der Anzeige (9). Die Anzeige (9) kann räumlich von dem Röntgengerät (2) getrennt sein. Der Rechner (8) kann vorzugsweise auch das Röntgengerät (2) steuern. Alternativ können separate Rechner für die Steuerung und die Rekonstruktion benutzt werden.

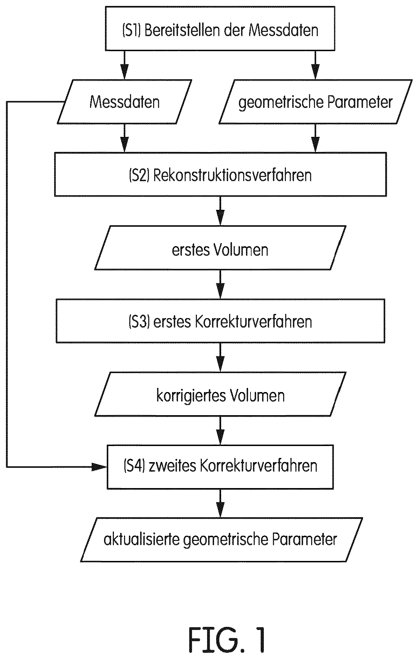

Abb.1 zeigt ein erfindungsgemäßes Flussdiagram. Das erfindungsgemäße Verfahren dient zur geometrischen Kalibrierung einer DVT-Aufnahme durch Aktualisierung der im Rekonstruktionsverfahren verwendeten geometrischen Parameter. Die Aktualisierung der geometrischen Parameter wird durch ein erstes Korrekturverfahren auf Basis maschinellen Lernens (ML) gestützt, indem das Ergebnis des ersten Korrekturverfahrens als Referenz für ein zweites Korrekturverfahren zur Parameterschätzung verwendet wird. Das zweite Korrekturverfahren zur Parameterschätzung bezieht die Messdaten der DVT-Aufnahme mit ein.

Das Verfahren umfasst die Schritte: (S 1) Bereitstellen der Messdaten der DVT Aufnahme und der geometrischen Parameter; (S2) Bereitstellen eines ersten Volumens durch Anwendung eines Rekonstruktionsverfahrens auf die bereitgestellten Messdaten und die geometrischen Parameter; (S3) Bereitstellung eines korrigierten Volumens durch Anwendung des ersten Korrekturverfahrens auf das erste Volumen; (S4) Bereitstellung von aktualisierten geometrischen Parametern durch Anwendung des zweiten Korrekturverfahrens auf die Messdaten und das korrigierte Volumen.

Die geometrischen Parameter oder aktualisierten geometrischen Parameter beschreiben die Projektionsgeometrie der DVT-Aufnahme relativ zum Patientenkopf. Die Projektionsgeometrie besteht vorzugsweise aus intrinsischen Parametern und extrinsischen Parametern, wobei die intrinsischen Parameter die relative Lage zwischen Röntgenstrahler (3) und Röntgendetektor (4) sowie deren Auflösung umfassen und die extrinsischen Parameter eine Transformation bestehend aus Rotation und Translation pro Projektionsbild umfassen.

Das neuronale Netzwerk (maschinelles Lernen (ML)) wird mit den folgenden Datenpaaren trainiert:
- Volumen einer DVT Aufnahme ohne Bewegungsartefakte und
- Volumen, die aus dem Sinogramm der DVT Aufnahme mit simulierter Patientenbewegung oder Gerätebewegung rekonstruiert wurden. Weitere Alternativen werden in dem Nachfolgenden Beschreibung später erläutert.

Es können optional Datenvorverarbeitungsschritte auf Bilddaten (Sinogramm oder Volumen) vor Schritt 3 und/oder Schritt 4 ergänzt werden, wie z.B. eine Kontrastverstärkung oder eine Hervorhebung relevanter Kanten oder eine Rauschunterdrückung, welche die Bilddaten so angleichen, dass das jeweilige Verfahren besser mit ihnen arbeitet.

Schritt (3) und Schritt (4) werden auf mindestens teilweise überlappende Bereiche des ersten oder des korrigierten Volumens angewendet, wobei der Schritt (3) vorzugsweise auf größeren Bereiche des ersten oder des korrigierten Volumens angewendet wird als Schritt (4).

Die geometrischen Parameter in Schritt (S 1) können vorzugsweise aus der angesteuerten und/oder erwarteten Gerätebewegung berechnet werden. Alternativ können sie durch eine geometrische Gerätekalibrierung ermittelt werden, welche zu einem früheren Zeitpunkt durchgeführt wurde. Bei dem Rekonstruktionsverfahren in Schritt (S2) kann es sich um ein Rekonstruktionsverfahren nach Feldkamp, nach Davis und Kress, oder eine iteratives Rekonstruktionsverfahren oder ein algebraisches Rekonstruktionsverfahren oder ein statistisches Rekonstruktionsverfahren handeln. In einer bevorzugten Ausführungsform umfasst das zweite Korrekturverfahren zur Parameterschätzung aus Schritt (S4) ein Registrierungsverfahren, welches die Messdaten mit den korrigierten Volumen aus Schritt (S3) registriert. Das Registrierungsverfahren bestimmt die aktualisierten geometrischen Parameter, welche die Aufnahme-Lagebeziehung zwischen den Messdaten und dem korrigierten Volumen beschreiben. In einer Ausführungsform erfolgt die Registrierung des Sinogramms durch die Erzeugung simulierter Sinogramme unter Anwendung der geometrischen Parameter und deren Vergleich mit dem Sinogramm durch Ähnlichkeitsmaße. Das Ähnlichkeitsmaß wird durch Variation der geometrischen Parameter maximiert. Bei der Erzeugung eines simulierten Sinogramms wird eine Vorwärtsprojektion des korrigierten Volumens durchgeführt unter Anwendung der geometrischen Parameter. Bei Anwendung der korrekten geometrischen Parameter ist die Ähnlichkeit zwischen dem simulierten Sinogramms und dem Sinogramm hoch. Da das korrigierte Volumen bereits eine Korrektur für Bewegungsartefakte enthält, berechnet das zweite Korrekturverfahren die passenden geometrischen Parameter für diese Korrektur. Die Kenntnis über die geometrischen Parameter ermöglicht nachfolgende Korrekturschritte oder eine Rekonstruktion unter Berücksichtigung der Rohdatendeckung bzw. Datentreue. In einer alternativen Ausführungsform umfasst das zweite Korrekturverfahren zur Parameterschätzung aus Schritt (S4) ein iteratives Rekonstruktionsverfahren, welches das korrigierte Volumen aus Schritt (S3) zur Regularisierung verwendet. Ein iteratives Rekonstruktionsverfahren variiert beliebige Rekonstruktionsparameter, wie zum Beispiel die geometrischen Parameter, und verbessert iterativ die Rohdatendeckung der Rekonstruktion. Eine Regularisierung umfasst die Differenz des korrigierten Volumens zum rekonstruierten Volumen als Strafterm. Die Verwendung der Regularisierung stabilisiert oder beschleunigt das zugrundeliegende Verfahren zur Parameteroptimierung.

In einer weiteren bevorzugtem Ausführungsform wird das zweite Korrekturverfahren zur Parameterschätzung aus Schritt (S4) nur auf einem oder mehreren selektierten Teilbereichen des korrigierten Volumens und/oder einem oder mehreren selektierten Teilbereichen der Messdaten ausgeführt. Teilbereiche des korrigierten Volumens können auch einzelne Volumenschichten sein. Die Selektion findet statt durch Vergleich des korrigierten und des ersten Volumens oder erfolgt alternativ direkt durch das erste Korrekturverfahren oder erfolgt alternativ durch eine manuelle Auswahl. Die Ausführung des zweiten Korrekturverfahrens auf selektierte Teilbereiche des korrigierten Volumens und/oder einen selektierten Teilbereich der Messdaten bietet die Vorteile, dass die Rechenzeit und der Ressourcenverbrauch des zweiten Korrekturverfahrens verringert werden. Zudem können so nicht-rigide Bewegungen lokal durch rigide Bewegungen angenähert werden. Die Selektion eines Teilbereichs des korrigierten Volumens kann durch Vergleich mit dem ersten Volumen erfolgen, indem Bereiche mit für die Korrektur signifikanten Änderungen ermittelt werden. Die Selektion der Teilbereiche der Messdaten kann durch zeitliche Unterabtastung, zeitliche Selektion der Projektionsbilder oder durch örtliche Selektion in den Projektionsbildern erfolgen. Hierzu können die selektierten Teilbereiche des korrigierten Volumens in das Sinogramm vorwärtsprojiziert werden unter Berücksichtigung der geometrischen Parameter. Die alternative Selektion der Teilbereiche durch das erste Korrekturverfahren hat den Vorteil, dass das ML Verfahren die Signifikanz der Änderungen im Volumen unabhängig von der Differenz der Volumen bestimmen kann. Auch die zeitliche Selektion der Projektionsbilder durch Auswertung der Bewegungsrichtung und des Bewegungszeitpunktes ist im Rahmen des ersten Korrekturverfahrens leicht realisierbar.

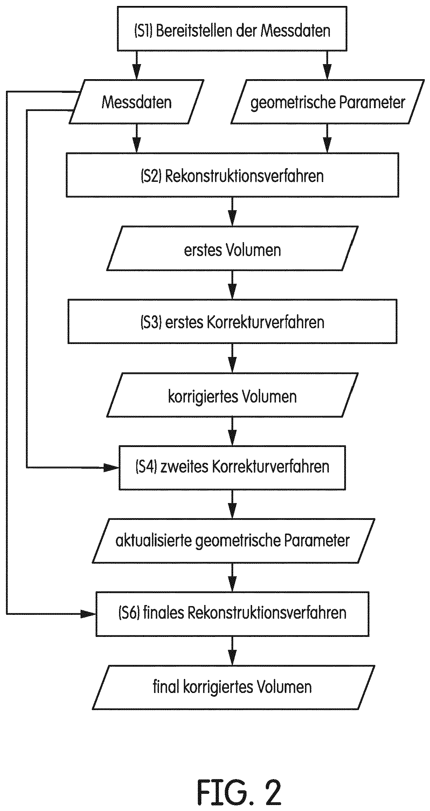

Abb.2 zeigt eine weitere bevorzugte Ausführungsform, in dem das Verfahren einen weiteren Schritt (S6) umfasst, zur Bereitstellung eines final korrigierten Volumens durch Anwendung eines finalen Rekonstruktionsverfahrens auf die Messdaten und die aktualisierten geometrischen Parameter aus dem Schritt (S4). Dieser Schritt (S6) erzeugt ein Volumen mit korrigierten Bewegungsartefakten unter Berücksichtigung der Rohdatendeckung.

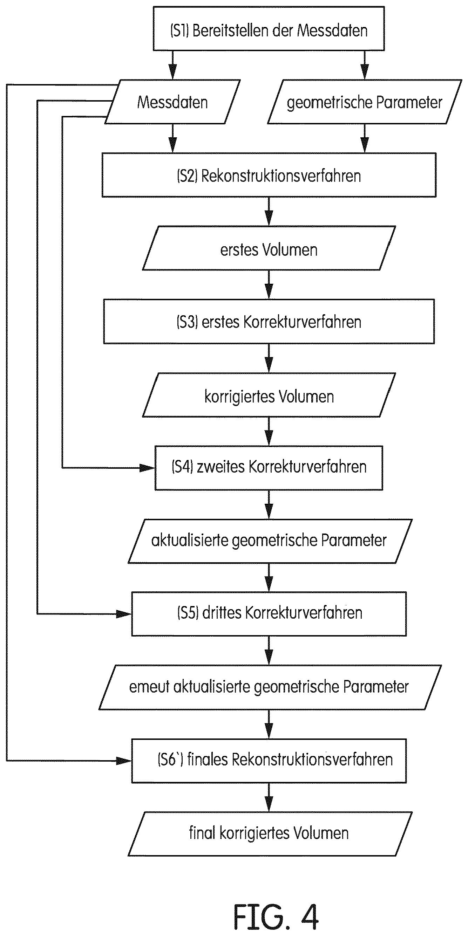

Abb.4 zeigt eine weitere *alternative* bevorzugte Ausführungsform. In dieser Ausführungsform umfasst das Verfahren einen Schritt (S5) zur Bereitstellung von erneut aktualisierten geometrischen Parametern durch Anwendung eines dritten Korrekturverfahrens zur Parameterschätzung auf die Messdaten und die aktualisierten geometrischen Parameter aus dem Schritt (S4). Und das Verfahren umfasst einen alternativen Schritt (S6') zur Bereitstellung eines final korrigierten Volumens durch Anwendung eines finalen Rekonstruktionsverfahrens auf die Messdaten und die erneut aktualisierten geometrischen Parameter aus dem Schritt (S5). Der Schritt (S5) ermöglicht es, mit einem weiteren Korrekturverfahren zur Parameterschätzung die geometrischen Parameter erneut zu verbessern und somit die initiale Schätzung des ersten Korrekturverfahrens zu verbessern. Dies kann in einer Ausführungsform folgende Schritte umfassen, welche vorzugsweise iterativ wiederholt werden bis ein Konvergenzkriterium erreicht ist: Rekonstruktion eines temporären Volumens mit geschätzter Projektionsgeometrie und Schätzung der Projektionsgeometrie durch Registrierung der Projektionsbilder des Sinogramms mit dem temporären Volumen. In einer Ausführungsform erfolgt die Registrierung des Sinogramms durch die Erzeugung simulierter Sinogramme unter Anwendung der geometrischen Parameter und deren Vergleich mit dem Sinogramm durch Ähnlichkeitsmaße. Das Ähnlichkeitsmaß wird durch Variation der geometrischen Parameter maximiert. Bei der Erzeugung eines simulierten Sinogramms wird eine Vorwärtsprojektion des korrigierten Volumens durchgeführt unter Anwendung der geometrischen Parameter. Der Schritt (S6') erzeugt ein final korrigiertes Volumen mit korrigierten Bewegungsartefakten unter Berücksichtigung der Rohdatendeckung.

Zusätzlich zu den Bilddaten können auch initiale geometrischen Parameter (z.B. Daten der aktuellen Gerätekalibrierung) als Eingabedaten den Verfahrensschritten (3)-(5) übergeben werden.

In weiteren bevorzugtem alternativen Ausführungsformen werden in dem finalen Rekonstruktionsverfahren aus Schritt (S6;S6') Teilbereiche des finalen Volumens erzeugt, und diese zu dem finalen Volumen zusammenfügt. Werden die Korrekturverfahren in Schritt (S4) und/oder Schritt (S5) auf mehreren Teilbereichen des Volumens oder der Messdaten ausgeführt, so wird pro Teilbereich ein Satz geometrischer Parameter bestimmt. Mit jedem Satz geometrischer Parameter kann ein Teilbereich des Volumens rekonstruiert werden. Die Teilbereiche des Volumens können im finalen Volumen zusammengeführt werden. Wenn die Teilbereiche des Volumens überlappen, können sie geeignet überblendet oder kombiniert werden.

Werden die Korrekturverfahren in Schritt (4) und/oder Schritt (5) auf einem oder mehreren selektierten Teilbereichen des Volumens oder der Messdaten ausgeführt, so wird pro selektiertem Teilbereich ein Satz geometrischer Parameter bestimmt. Die Sätze geometrischer Parameter können interpoliert oder extrapoliert werden, um geometrische Parameter für die nicht selektierten Teilbereiche des Volumens oder der Messdaten zu bestimmen. Hierbei können die Teilbereiche im Volumen selektiert und in den Messdaten interpoliert oder extrapoliert werden und andersherum.

In weiteren bevorzugten alternativen Ausführungsformen werden in dem finalen Rekonstruktionsverfahren aus Schritt (S6;S6') die aktualisierten geometrischen Parameter der Teilbereiche aus Schritt (S4) oder die erneut aktualisierten geometrischen Parameter der Teilbereiche aus Schritt (S5) interpoliert oder extrapoliert, wobei die Interpolations- oder Extrapolationsgewichte aus der relativen Lage der nicht selektierten Teilbereiche im Volumen zu den selektierten Teilbereichen im Volumen bestimmt werden..

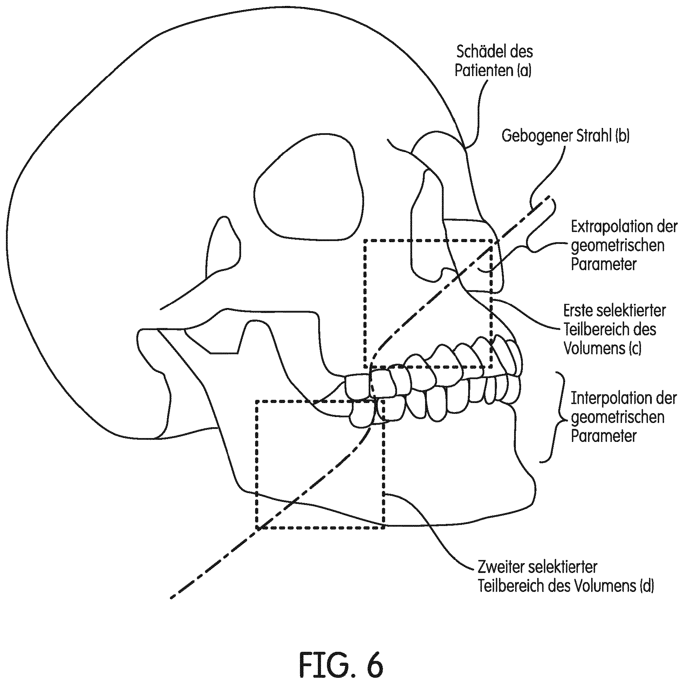

In einer Ausführungsform mit mehreren selektierten Teilbereichen können die geometrischen Parameter örtlich im Volumen definiert werden. Somit können pro Voxel geometrische Parameter bestimmt werden, so dass pro Voxel und Projektionsbild eine Transformation beschrieben wird. Dies ermöglicht in der Rekonstruktion die Realisierung nicht gerader, bzw. gebogener Strahlen, so dass nicht-rigide Bewegungen in der Rekonstruktion berücksichtigt werden können. Die gebogenen Strahlen kann man so verstehen, dass sie jeweils eine Zusammensetzung von geraden Teilstücken sind, die sich aus der Bewegung oder Verformung der Patientenanatomie während der Aufnahme ergeben. Abb. 6 zeigt einen gebogenen Strahl (b) durch den Schädel des Patienten (a), welcher den Pfad der Röntgenstrahlen während der Aufnahme für ein starres Volumen in der Rekonstruktion darstellt. Die geometrischen Parameter werden für die Rekonstruktion zwischen den zwei selektierten Teilbereichen des Volumens (c,d) interpoliert und außerhalb der zwei selektierten Teilbereiche des Volumens (c,d) extrapoliert.

In weiteren alternativen bevorzugten Ausführungsformen werden in dem finalen Rekonstruktionsverfahren aus Schritt (S6;S6') die aktualisierten geometrischen Parameter der Teilbereiche aus Schritt (S4) oder die erneut aktualisierten geometrischen Parameter der Teilbereiche aus Schritt (S5) in den Messdaten interpoliert oder extrapoliert, wobei die Interpolations- oder Extrapolationsgewichte aus der relativen örtlichen oder zeitlichen Lage der nicht selektierten Teilbereiche der Messdaten zu den selektierten Teilbereichen der Messdaten bestimmt werden.

In einer Ausführungsform können die geometrischen Parameter zeitlich in den Messdaten interpoliert oder extrapoliert werden, indem die geometrischen Parameter in der zeitlichen Dimension des Sinogramms interpoliert oder extrapoliert werden. In einer weiteren Ausführungsform können die geometrischen Parameter örtlich in den Messdaten interpoliert oder extrapoliert werden, indem die geometrischen Parameter in den örtlichen Dimensionen des Sinogramms interpoliert oder extrapoliert werden.

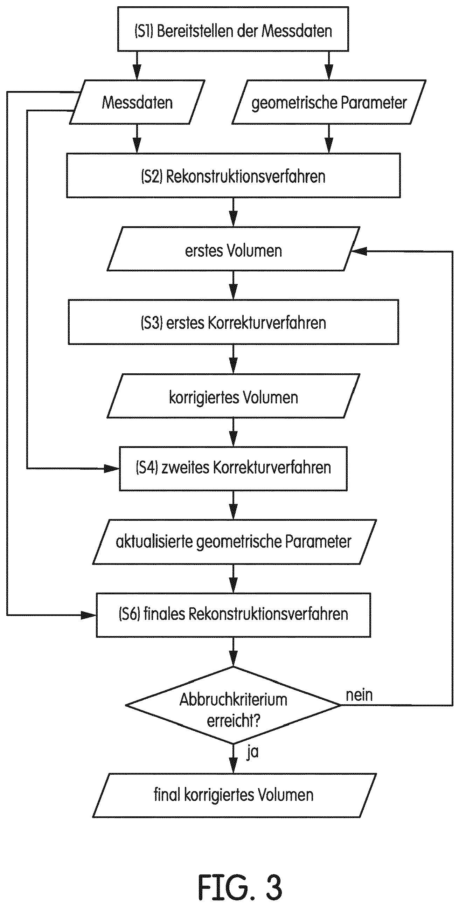

Abb.3 zeigt eine weitere alternative bevorzugte Ausführungsform. In dieser Ausführungsform werden die Schritte (S3), (S4) und (S6) ein- oder mehrfach wiederholt bzw. auch iterativ durchgeführt. Die iterative Wiederholung der genannten Schritte erhöht die Korrektheit, Genauigkeit und/oder Konvergenz der Korrekturverfahren, da die Korrekturverfahren bei Eingabedaten mit einem kleinen Fehler in der Regel zu einem besseren Endergebnis führen als bei Eingabedaten mit einem großen Fehler. Die iterative Wiederholung wird abgebrochen, wenn ein Konvergenzkriterium erreicht ist. Mögliche Konvergenzkriterien sind: a) ob die Änderung der Projektionsgeometrie kleiner als ein Schwellwert ist; b) ob die Änderung im finalen Volumen kleiner als ein Schwellwert ist; c) ob die Anzahl der Iterationsschritte größer als ein Schwellwert ist; d) ob die Rechenzeit größer als ein Schwellwert ist.

In weiteren bevorzugten alternativen Ausführungsformen werden die Schritte (S3) bis (S6) ein- oder mehrfach wiederholt bzw. auch iterativ durchgeführt werden. Die iterative Wiederholung der genannten Schritte erhöht die Korrektheit, Genauigkeit und/oder Konvergenz der Korrekturverfahren, da die Korrekturverfahren bei Eingabedaten mit einem kleinen Fehler in der Regel zu einem besseren Endergebnis führen als bei Eingabedaten mit einem großen Fehler. Die iterative Wiederholung wird abgebrochen, wenn ein Konvergenzkriterium erreicht ist. Mögliche Konvergenzkriterien sind: a) ob die Änderung der Projektionsgeometrie kleiner als ein Schwellwert ist; b) ob die Änderung im finalen Volumen kleiner als ein Schwellwert ist; c) ob die Anzahl der Iterationsschritte größer als ein Schwellwert ist; d) ob die Rechenzeit größer als ein Schwellwert ist.

Gemäß der vorliegenden Erfindung können die Datensätze, die durch die oben aufgeführte Ausführungsformen erzeugt werden, zur Visualisierung, insbesondere für diagnostische Zwecke, einem Arzt vorgelegt werden, vorzugsweise mittels einer Anzeige (9) oder eines Ausdrucks.

In einer bevorzugten Ausführungsform wird das erste Korrekturverfahren als Bild-zu-Bild Transformation ausgeführt, hier speziell als Volumen-zu-Volumen Transformation. Das Training des ML-Netzwerkes erfolgt mit entsprechenden Datenpaaren bestehend aus je einem Volumen mit Bewegungsartefakten und einem Volumen ohne Bewegungsartefakte des gleichen aufgenommenen Messobjekts. Bei dem Messobjekt kann es sich um einen menschlichen Schädel oder technische Prüfkörper des dentalen DVT-Röntgengeräts oder Phantomen mit anatomischen Zahn- und Schädelstrukturen handeln. Die Datenpaare können durch Simulation erzeugt werden. In einer Ausführungsform erfolgt die Simulation des Volumens mit Bewegungsartefakten durch Variation der Rekonstruktionsparameter einer DVT-Aufnahme ohne Bewegungsartefakte. In einer anderen Ausführungsform erfolgt die Simulation des Volumens mit Bewegungsartefakten durch Erzeugung simulierter Sinogramme aus einem Volumen ohne Bewegungsartefakte unter Variation der Projektionsparameter und anschließender Volumenrekonstruktion aus dem simulierten Sinogramm. In einer weiteren Ausführungsform wird das Volumen ohne Bewegungsartefakte mithilfe eines konventionellen Verfahrens zur Parameterschätzung aus einer DVT-Aufnahme mit Bewegungsartefakten berechnet. In einer weiteren Ausführungsform werden die Volumen mit Bewegungsartefakten durch Bewegung des Messobjekts während der Aufnahme erzeugt. Die Bewegung des Messobjekts kann absichtlich oder gezielt erfolgen.

Es gibt eine Vielzahl konventioneller Verfahren zur Parameterschätzung in der Literatur. Im Schritt S4 wird ein Verfahren zur Parameterschätzung angewandt, welches aus einem gegebenen Volumen und gegebenen Messdaten aktualisierte geometrische Parameter berechnet. In einer bevorzugten Ausführungsform erfolgt die Schätzung der geometrischen Parameter durch Bewertung der Differenzen zwischen einer Vorwärtsprojektionen des gegebenen Volumens und den Messdaten. Dies entspricht einer Bewertung der Datentreue, beziehungsweise einem Registrierungsverfahren. In einer weiteren Ausführungsform erfolgt die Schätzung der geometrischen Parameter im Rahmen eines iterativen Rekonstruktionsverfahrens, welches in einem Strafterm Vorwissen oder Randbedingungen berücksichtigt. So kann die Differenz zu dem gegebenen Volumen als Regularisierung eingehen und/oder die Differenz zu initial angenommenen geometrischen Parametern. Hierbei kommen Ähnlichkeitsmaße zum Einsatz, welche zum Beispiel die Histogramme, die Gradienten und/oder die Grauwerte der Volumen vergleichen.

In Schritt S5 wird ein Verfahren zur Parameterschätzung angewandt, welches aus gegebenen geometrischen Parametern und gegebenen Messdaten aktualisierte geometrische Parameter berechnet. In einer Ausführungsform erfolgt die Schätzung der geometrischen Parameter durch Verbesserung einer Metrik zur Bewertung der Bildqualität des Volumens. In einer weiteren Ausführungsform erfolgt die Schätzung der geometrischen Parameter mithilfe der Auswertung von Datenkonsistenzbedingungen im Sinogramm. In einer weiteren Ausführungsform erfolgt die Schätzung der geometrischen Parameter mithilfe eines iterativen Rekonstruktionsverfahren, welches die Differenz zwischen den Vorwärtsprojektionen eines rekonstruierten Volumens und den Messdaten iterativ verringert. In einer weiteren Ausführungsform erfolgt die Schätzung der geometrischen Parameter mithilfe eines iterativen Registrierungsverfahrens, welches iterativ ein Volumen mit den aktuellen geometrischen Parametern rekonstruiert und anschließen die geometrischen Parameter durch Registrierung der Messdaten mit dem rekonstruierten Volumen schätzt.

## Patentansprüche

1. Computer-implementiertes Verfahren zur geometrischen Kalibrierung einer DVT-Aufnahme durch Aktualisierung der im Rekonstruktionsverfahren verwendeten geometrischen Parameter,
wobei die Aktualisierung der geometrischen Parameter durch ein erstes Korrekturverfahren auf Basis maschinelles Lernens (ML) gestützt wird, indem ein durch das erste Korrekturverfahren korrigierte Volumen als Referenz für ein zweites Korrekturverfahren zur geometrischen Parameterschätzung verwendet wird, und
wobei das maschinelle Lernen des ersten Korrekturverfahrens mit den folgenden Datenpaaren trainiert wird: Volumen einer DVT Aufnahme ohne Bewegungsartefakte, und
Volumen einer DVT Aufnahme mit Bewegungsartefakten des gleichen aufgenommenen Messobjekts, und
wobei das zweite Korrekturverfahren zur Parameterschätzung die Messdaten der DVT-Aufnahme mit einbezieht, umfassend folgende Schritte:
(S1) Bereitstellen der Messdaten der DVT Aufnahme und der geometrischen Parameter, wobei die Messdaten ein Sinogramm der DVT-Aufnahme bestehend aus Röntgenprojektionen umfassen, und wobei die geometrischen Parameter die Projektionsgeometrie der DVT-Aufnahme beschreiben;
(S2) Bereitstellen eines ersten Volumens durch Anwendung eines Rekonstruktionsverfahrens auf die bereitgestellten Messdaten und die geometrischen Parameter;
(S3) Bereitstellung eines korrigierten Volumens durch Anwendung des ersten Korrekturverfahrens auf Basis maschinelles Lernens (ML) auf das erste Volumen;
(S4) Bereitstellung von aktualisierten geometrischen Parametern durch Anwendung des zweiten Korrekturverfahrens auf die Messdaten und das korrigierte Volumen.

2. Verfahren nach Anspruch 1, wobei das zweite Korrekturverfahren zur Parameterschätzung aus Schritt (S4) ein Registrierungsverfahren umfasst, welches die Messdaten mit den korrigierten Volumen aus Schritt (S3) registriert.

3. Verfahren nach Anspruch 1, wobei das zweite Korrekturverfahren zur Parameterschätzung aus Schritt (S4) ein iteratives Rekonstruktionsverfahren umfasst, welches das korrigierte Volumen aus Schritt (S3) zur Regularisierung verwendet.

4. Verfahren nach einem der vorangegangenen Ansprüche, wobei
das zweite Korrekturverfahren zur Parameterschätzung aus Schritt (S4) nur auf einem selektierten Teilbereich des korrigierten Volumens und/oder einem selektierten Teilbereich der Messdaten ausgeführt wird,
wobei die Selektion durch Vergleich des korrigierten und des ersten Volumens stattfindet, oder alternativ direkt durch das erste Korrekturverfahren erfolgt.

5. Verfahren nach einem der vorangegangenen Ansprüche, umfassend folgenden Schritt:
(S6) Bereitstellung eines final korrigierten Volumens durch Anwendung eines finalen Rekonstruktionsverfahrens auf die Messdaten und die aktualisierten geometrischen Parameter aus dem Schritt (S4).

6. Verfahren nach einem der Ansprühe 1-4, umfassend folgende Schritte,
(S5) Bereitstellung von erneut aktualisierten geometrischen Parametern durch Anwendung eines dritten Korrekturverfahrens zur Parameterschätzung auf die Messdaten und die aktualisierten geometrischen Parameter aus dem Schritt (S4);
(S6') Bereitstellung eines final korrigierten Volumens durch Anwendung eines finalen Rekonstruktionsverfahrens auf die Messdaten und die erneut aktualisierten geometrischen Parameter aus dem Schritt (S5).

7. Verfahren nach Anspruch 4 und einem der Ansprüche 5 oder 6, wobei
das finale Rekonstruktionsverfahren aus Schritt (S6;S6') Teilbereiche des finalen Volumens erzeugt, und diese zu dem finalen Volumen zusammenfügt.

8. Verfahren nach Anspruch 4 und einem der Ansprüche 5 oder 6, wobei
das finale Rekonstruktionsverfahren aus Schritt (S6) die aktualisierten geometrischen Parameter der Teilbereiche aus Schritt (S4) oder die erneut aktualisierten geometrischen Parameter der Teilbereiche aus Schritt (S5) interpoliert oder extrapoliert und wobei die Interpolations- oder Extrapolationsgewichte aus der relativen Lage der nicht selektierten Teilbereiche im Volumen zu den selektierten Teilbereichen im Volumen bestimmt werden.

9. Verfahren nach Anspruch 4 und einem der Ansprüche 5 oder 6, wobei
das finale Rekonstruktionsverfahren aus Schritt (S6) die aktualisierten geometrischen Parameter der Teilbereiche aus Schritt (S4) oder die erneut aktualisierten geometrischen Parameter der Teilbereiche aus Schritt (S5) interpoliert oder extrapoliert und wobei die Interpolations- oder Extrapolationsgewichte aus der relativen örtlichen oder zeitlichen Lage der nicht selektierten Teilbereiche der Messdaten zu den selektierten Teilbereichen der Messdaten bestimmt werden.

10. Verfahren nach den Ansprüchen 5 bis 9, wobei die Schritte (S3), (S4) und (S6), oder (S3) bis (S6) ein- oder mehrfach wiederholt bzw. auch iterativ durchgeführt werden.

11. Computerprogramm umfassend computerlesbare Code, das wenn es von einem computergestützten DVT-System (1) ausgeführt wird und dieses veranlasst, die Verfahrensschritte einer der vorhergehenden Verfahrensansprüche auszuführen.

12. Computergestütztes DVT-System (1) umfassend ein Röntgengerät (2) für eine dentale Patientenaufnahme und eine Recheneinheit (8) die zur Ausführung des Computerprogramms nach Anspruch 11 konfiguriert ist.

13. Die Verwendung von einem Datensatz zur Visualisierung der durch einen der vorangegangen Ansprüche 1 bis 10 zur Verfügung gestellt wurde.

## Claims

1. Computer-implemented method for the geometric calibration of a CBCT image by updating the geometric parameters used in the reconstruction method, wherein the updating of the geometric parameters is supported by a first correction method based on machine learning (ML) by using a volume corrected by the first correction method as a reference for a second correction method for geometric parameter estimation, and
wherein the machine learning of the first correction method is trained having the following data pairs: volume of a CBCT image without motion artifacts, and
volume of a CBCT image having motion artifacts of the same recorded object, and
wherein the second correction method for parameter estimation includes the measurement data of the CBCT image, comprising the following steps:
(S1) providing the measurement data of the CBCT image and the geometric parameters, wherein the measurement data comprise a sinogram of the CBCT image consisting of X-ray projections, and wherein the geometric parameters describe the projection geometry of the CBCT image;
(S2) providing a first volume by applying a reconstruction method to the provided measurement data and the geometric parameters;
(S3) providing a corrected volume by applying the first correction method based on machine learning (ML) to the first volume;
(S4) providing updated geometric parameters by applying the second correction method to the measurement data and the corrected volume.

2. Method according to claim 1, wherein the second correction method for parameter estimation from step (S4) comprises a registration method which registers the measurement data having the corrected volumes from step (S3).

3. Method according to claim 1, wherein the second correction method for parameter estimation from step (S4) comprises an iterative reconstruction method which uses the corrected volume from step (S3) for regularization.

4. Method according to any of the preceding claims, wherein the second correction method for parameter estimation from step (S4) is carried out only on one selected partial area of the corrected volume and/or one selected partial area of the measurement data,
wherein the selection takes place by comparing the corrected and the first volume, or alternatively directly by the first correction method.

5. Method according to any of the preceding claims, comprising the following step:
(S6) providing a final corrected volume by applying a final reconstruction method to the measurement data and the updated geometric parameters from step (S4).

6. Method according to any of claims 1 to 4, comprising the following steps, (S5) providing re-updated geometric parameters by applying a third correction method for parameter estimation to the measurement data and the updated geometric parameters from step (S4);
(S6') providing a final corrected volume by applying a final reconstruction method to the measurement data and the re-updated geometric parameters from step (S5).

7. Method according to claim 4 and any of claim 5 or 6, wherein the final reconstruction method from step (S6;S6') generates partial regions of the final volume and combines them to form the final volume.

8. Method according to claim 4 and any of claim 5 or 6, wherein the final reconstruction method from step (S6) interpolates or extrapolates the updated geometric parameters of the sub-regions from step (S4) or the re-updated geometric parameters of the sub-regions from step (S5), and wherein the interpolation or extrapolation weights are determined from the relative position of the non-selected sub-regions in the volume to the selected sub-regions in the volume.

9. Method according to claim 4 and any of claim 5 or 6, wherein
the final reconstruction method from step (S6) interpolates or extrapolates the updated geometric parameters of the sub-regions from step (S4) or the re-updated geometric parameters of the sub-regions from step (S5), and wherein the interpolation or extrapolation weights are determined from the relative local or temporal position of the non-selected sub-regions of the measurement data to the selected sub-regions of the measurement data.

10. Method according to claims 5 to 9, wherein the steps (S3), (S4) and (S6), or (S3) to (S6) are repeated one or more times or are also carried out iteratively.

11. Computer program comprising computer-readable code which when it is executed by a computerized CBCT system (1) and prompts said system to execute the method steps of any one of the preceding method claims.

12. Computerized CBCT system (1) comprising an X-ray device (2) for dental patient imaging and a computing unit (8) which is configured to execute the computer program according to claim 11.

13. The use of a data set for visualization supplied by any of the preceding claims 1 to 10.

## Revendications

1. Procédé implémenté par ordinateur pour l'étalonnage géométrique d'une tomographie numérique de volume, TNV, par mise à jour des paramètres géométriques utilisés dans le procédé de reconstruction,
la mise à jour des paramètres géométriques étant assistée par un premier procédé de correction basé sur l'apprentissage automatique (ML), en utilisant un volume corrigé par le premier procédé de correction comme référence pour un deuxième procédé de correction pour l'estimation des paramètres géométriques, et
l'apprentissage automatique du premier procédé de correction étant entraîné avec les paires de données suivantes : le volume d'une TNV sans artefacts de mouvement, et le volume d'une TNV avec artefacts de mouvement du même objet de mesure enregistré, et
le deuxième procédé de correction pour l'estimation des paramètres incluant les données de mesure de la TNV, comprenant les étapes suivantes :
(S1) Mise à disposition des données de mesure de la TNV et des paramètres géométriques, les données de mesure comprenant un sinogramme de la TNV constitué de projections de rayons X, et les paramètres géométriques décrivant la géométrie de projection de la TNV ;
(S2) Mise à disposition d'un premier volume en appliquant un procédé de reconstruction aux données de mesure et aux paramètres géométriques mis à disposition ;
(S3) Mise à disposition d'un volume corrigé en appliquant le premier procédé de correction basé sur l'apprentissage automatique (ML) au premier volume ;
(S4) Mise à disposition des paramètres géométriques mis à jour en appliquant le deuxième procédé de correction aux données de mesure et au volume corrigé.

2. Procédé selon la revendication 1, dans lequel le deuxième procédé de correction pour l'estimation des paramètres de l'étape (S4) comprend un procédé d'enregistrement qui enregistre les données de mesure avec les volumes corrigés de l'étape (S3).

3. Procédé selon la revendication 1, dans lequel le deuxième procédé de correction pour l'estimation des paramètres de l'étape (S4) comprend un procédé de reconstruction itératif qui utilise le volume corrigé de l'étape (S3) pour la régularisation.

4. Procédé selon l'une des revendications précédentes,
le deuxième procédé de correction pour l'estimation de paramètres de l'étape (S4) étant exécuté uniquement sur une zone partielle sélectionnée du volume corrigé et/ou une zone partielle sélectionnée des données de mesure,
la sélection s'effectuant par comparaison du volume corrigé et du premier volume, ou en variante directement par le premier procédé de correction.

5. Procédé selon l'une des revendications précédentes, comprenant l'étape suivante :
(S6) Mise à disposition d'un volume corrigé final en appliquant un procédé de reconstruction finale aux données de mesure et aux paramètres géométriques mis à jour de l'étape (S4).

6. Procédé selon l'une des revendications 1 à 4, comprenant les étapes de
(S5) Mise à disposition des paramètres géométriques à nouveau mis à jour en appliquant un troisième procédé de correction pour estimer les paramètres aux données de mesure et aux paramètres géométriques mis à jour de l'étape (S4) ;
(S6') Mise à disposition d'un volume corrigé final en appliquant un procédé de reconstruction finale aux données de mesure et aux paramètres géométriques mis à jour à nouveau de l'étape (S5).

7. Procédé selon la revendication 4 et l'une des revendications 5 ou 6,
le procédé de reconstruction finale de l'étape (S6;S6') générant des zones partielles du volume final, et les assemblant pour former le volume final.

8. Procédé selon la revendication 4 et l'une des revendications 5 ou 6,
le procédé de reconstruction finale de l'étape (S6) interpolant ou extrapolant les paramètres géométriques mis à jour des zones partielles de l'étape (S4) ou les paramètres géométriques à nouveau mis à jour des zones partielles de l'étape (S5) et les poids d'interpolation ou d'extrapolation étant déterminés à partir de la position relative des zones partielles non sélectionnées dans le volume par rapport aux zones partielles sélectionnées dans le volume.

9. Procédé selon la revendication 4 et l'une des revendications 5 ou 6,
le procédé de reconstruction finale de l'étape (S6) interpolant ou extrapolant les paramètres géométriques mis à jour des zones partielles de l'étape (S4) ou les paramètres géométriques à nouveau mis à jour des zones partielles de l'étape (S5) et les poids d'interpolation ou d'extrapolation étant déterminés à partir de la position locale ou temporelle relative des zones partielles non sélectionnées des données de mesure par rapport aux zones partielles sélectionnées des données de mesure.

10. Procédé selon les revendications 5 à 9, les étapes (S3), (S4) et (S6), ou (S3) à (S6), étant répétées une ou plusieurs fois ou étant également exécutées de manière itérative.

11. Programme informatique comprenant un code lisible par ordinateur qui, lorsqu'il est exécuté par un système TNV assisté par ordinateur (1), amène ce dernier à exécuter les étapes de procédé de l'une des revendications de procédé précédentes.

12. Système TNV assisté par ordinateur (1) comprenant un appareil de radiographie (2) pour une prise de vue dentaire du patient et une unité de calcul (8) qui est configurée pour exécuter le programme informatique selon la revendication 11.

13. Utilisation d'un ensemble de données pour la visualisation fournie par l'une des revendications précédentes 1 à 10.
